# EUROPEAN PATENT SPECIFICATION

(11) **EP 3 110 486 B1**
(45) Date of publication and mention of the grant of the patent: **08.01.2020**
(21) Application number: 15704783.8
(22) Date of filing: 13.02.2015
(51) Int. Cl.: A61M 16/04, A61M 16/08, A61M 16/00

(54) **LARYNGEAL MASK**
LARYNXMASKE
MASQUE LARYNGÉ

(30) Priority: 25.02.2014 ES 201430257 P
(43) Date of publication of application: 04.01.2017
(73) Proprietor: Medcom Flow S.A., 08029 Barcelona (ES)
(72) Inventor: ACHA GANDARIAS, Pedro, E-08029 Barcelona (ES); SAGALÉS MAÑAS, Juan, E-08029 Barcelona (ES); ROCA DE VIÑALS DELGADO, Alejandro, E-08029 Barcelona (ES); CALAF ALCALDE, Alberto, E-08029 Barcelona (ES)
(74) Representative: Elzaburu S.L.P.
(86) International application number: PCT/EP2015/053078
(87) International publication number: WO 2015/128207

(56) References cited:
- CN-A- 103 223 204
- US-A1- 2008 099 026
- US-A1- 2009 229 601
- US-A1- 2010 319 704
- US-A1- 2013 081 616
- US-A1- 2013 291 868
- US-A1- 2013 324 798
- US-B2- 7 004 169

## Description

### Technical field

The present invention is related to the field of ventilation of the human or animal body.

### Description of the invention

The modality of breathing support with continuous oxygen positive pressure makes it possible to increase alveolar ventilation of the patient, by creating a positive pressure of oxygen in the patient's airways which can be constant or not constant and which will be the minimum pressure that the patient will obtain in their airways conducts by this means during their phase of full exhalation.

To date this continuous oxygen positive pressure was created in the accesses to the patient's airways, i.e. in the mouth or nose, without the need for accessing the internal airway artificially, as distinct from what occurs in the case of endotracheal intubation. Up until now, in ventilation using continuous oxygen positive pressure facial mask or nasal prong type devices are used which apply some pressure on the skin of the patient face to try to avoid oxygen leaks and for all the continuous oxygen positive pressure to try to reach the patient's airways in its totality , but with the big problem that the continuous oxygen positive pressure administration also goes to the patients stomach creating expansion, pain and risk of vomiting of the stomach secretions that can go to the lungs and develop a high risky aspiration pneumonia that can kill the patient. For this reason, because until now continuous positive pressure was not applied invasively inside the airway, it started to be called non-invasive mechanical ventilation.

There are several devices on the market which create this continuous oxygen positive pressure on the accesses to the airways such as the mouth or nose. This technique consists of applying a continuous air or oxygen positive pressure (measured in cm of water) above that of atmospheric pressure, thus increasing the volume of the lungs and increasing oxygenation, which helps the patient to breathe spontaneously and to have a continuous positive pressure always, even in the respiratory phase of complete exhalation. This technique is extensively used, among others, in patients with obstructive sleep apnea.

The application of continuous positive pressure to the airway in a non-invasive manner dates back to 1930, when Alvan Barach demonstrated that continuous oxygen positive pressure in the airways was useful in the treatment of acute pulmonary oedema, but it was Gregory in 1971 who gave it its name and describes it initially, applying it to respiratory distress in newborns. Shortly afterwards, Civetta and Falke described its advantages in adults with acute respiratory failure back in 1972.

In comparison with endotracheal intubation, continuous oxygen positive pressure presents, among others, the advantages of a lower risk of complications, allows the patient to be awake and to cough and keep the airway reflexes and eliminate secretions, improves the quality of sleep and facilitates the fact that the patient uses their respiratory muscles to avoid muscle atrophy.

On a separate note, on other more severe illnesses it is also necessary to put the patient to sleep and to resort to endotracheal intubation, for example in interventions combined with anaesthesia, for example for patients who require intubation with an endotracheal tube to allow for their oxygenation, or to resort to artificial ventilation with a laryngeal mask device with the patient also asleep.

Prior art devices are disclosed in US 2008/0099026 A1, CN 103 223 204 A, US 2013/0291868 A1, US 2010/0319704 A1, US 2013/0081616 A1, US 2009/0229601 A1, US 2013/0324798 A1, US 7 004 169 B2.

However, there are cases like in the present invention, in which it would be desirable to have a device that could act independently as a standard laryngeal mask for standard mechanical ventilation and also as a laryngeal mask for continuous oxygen positive pressure in a patient in spontaneous breathing. This device would be advantageous, for example, in cases wherein the pulmonary pathology of the patient is more severe and requires a greater contribution of oxygen at positive pressure than a facial mask or prongs, or in patients in whom it is necessary to conduct a diagnostic or therapeutic test via the airways, wherein the oxygen supply is reduced as a consequence of this as in the case when using probes, fiberscopes, etc., and wherein at the same time it is necessary to supply oxygen to the patient, but without the high risk of administering also continuous oxygen positive pressure to the stomach where you can create expansion, dilatation, pain and vomiting with risky bronchoaspiration pneumonia.

The device according to the invention aims to respond to this need, by providing a device which combines the performance of a standard laryngeal mask device with a laryngeal mask for administering continuous oxygen positive pressure. A device of this type helps to solve the aforementioned problems and thus achieve the desired objectives.

The solution to this problem is based on the fact that the inventor has found that such a device can be built on the basis of a standard laryngeal mask that is provided with at least two tubes where both of them can work for the inlet and/or outlet of oxygen or gases, to and from the inner airways of the patent. Both tubes can have the same or different diameter. When the device is intended to work as a standard laryngeal mask, only one of these tubes remains open for the inlet and outlet of the oxygen or gases, the other one remaining closed. Under these circumstances, the device works in conventional ventilation mode as a standard laryngeal mask, and through the open tube oxygen or gases are alternatively forced in and out of the airways of the patient, so inhaled and exhaled gases are expelled in and out of the patient's airways. On the other hand, when the device is intended to work in continuous oxygen positive pressure mode, both tubes remain open, optionally to a variable extent. Under these circumstances, a continuous flow of air or oxygen is applied to one of the tubes from an external oxygen supply system, whereas the exhaled air or gases are allowed to exit through the other tube. Through the adequate variable regulation of the flow of air or oxygen applied to one tube, and the variable opening of the other tube, an adequate regulation of the amount of oxygen introduced into the patient's airways and the exhaled gases is achieved. Such circumstances create and keep a residual of positive oxygen pressure in the dome of our laryngeal mask and in the patient's airway. By varying or changing the inside flow supply and by changing the variable diameter of both tubes we can achieve a residual positive pressure than can be higher or lower.

In consequence, the invention is directed at a laryngeal mask according to claim 1 has the following components:
- a dome (2) having a cavity (2a), which dome (2) is attached to a laryngeal ring (3) for sealing the patient's internal airways;
- at least one oxygen tube (4) that connects to the cavity (2a) of the dome (2), for allowing oxygen or gases to enter or leave the patient's internal airways; and
- at least one laryngeal aspiration tube (5), different from the oxygen tube (4), that also connects to the cavity (2a) of the dome (2), for allowing air and/or secretions to leave the patient's internal airways.

Even though all the tubes can vary or change in diameter independently of each other and do not need to have the same or different diameter, however the diameter of the laryngeal aspiration tube (5) is smaller than the diameter of the oxygen tube (4). The diameter of the laryngeal aspiration tube (5) is between 20 and 70% the diameter of the oxygen tube (4); in an even more preferred embodiment, the diameter of the laryngeal aspiration tube (5) is between 40 and 65% the diameter of the oxygen tube (4) and in a yet more preferred embodiment, the diameter of the laryngeal aspiration tube (5) is about 60% the diameter of the oxygen tube (5). In a very convenient embodiment, the inner diameter of the laryngeal aspiration tube (5) is 5 mm and the inner diameter of the oxygen tube (4) is 8.5 mm. In this way, through the use of a standard oxygen supply like a ventury flow system, with an oxygen flow of about 10 litres/minute we easily achieve or create a residual oxygen positive pressure in the internal airways of the patient of around 5 cm of water, which is an optimum value for operation under the conditions of continuous positive oxygen pressure. However, the same or different oxygen flow can likewise be achieved by other means, e.g. by an adequate regulation of the oxygen flow in the oxygen supply device, in combination with oxygen and laryngeal aspiration tubes of the same or different diameters in different combinations.

Also in preferred embodiments, the laryngeal mask additionally may comprise any of the following additional tubes in any combination:
- A gastric aspiration tube (6) that, contrary to the oxygen tube (4) and the laryngeal aspiration tube (5), does not connect to the cavity of the dome but it connects directly to the laryngeal tube orifice (3a) located at the distal portion of the laryngeal ring (3), usually passing through or below the cavity of the dome, to extract the excess air or oxygen accumulated in the patient's stomach by the administration of a continuous oxygen positive pressure and extract secretions accumulated, which could lead to gastric bloating and vomiting and aspiration pneumonia therein; and
- A tube (7) that also connects to the cavity (2a) of the dome (2) and is connected to a pressure-measuring device for measuring the pressure existing inside the dome of our laryngeal mask.

In one advantageous embodiment, the device according to the invention is provided with the oxygen tube (4) and the laryngeal aspiration tube (5), plus the other two aforesaid additional tubes. However, the invention is not limited to the existence of the four tubes necessarily, and it is possible to have at least the oxygen tube (4) and the laryngeal aspiration tube (5) plus any combination of any other tubes depending on the needs of the patient and/or of the doctor who is carrying out the operation.

In another preferred embodiment, the tube or tubes present in the device are joined by an additional piece which surrounds them and keeps them joined, this piece may be additionally rigid and also have the function of preventing the patient from biting the tubes making them collapse and close.

Each one of these tubes can be provided with respective caps or plugs that allows the tubes to be close or open in a variable degree which allow our laryngeal mask to be used in any of the two ventilation modes (as a standard laryngeal mask or as a continuous oxygen positive pressure laryngeal mask) and, by closing or opening or plugging or unplugging the caps on the tubes, or by joining them to other devices. To that end, the caps or plugs can have a variable opening. A cap having a variable opening is particularly useful for the laryngeal aspiration tube (5) because, in this way, the positive oxygen pressure kept residual inside the laryngeal mask dome or the patient's internal airways can be easily regulated, without the need to adjust the oxygen flow from the oxygen supply device. That, by the way, can also be adjusted to increase or decrease the positive oxygen pressure kept residual inside the laryngeal mask dome or the patient's internal airways.

### Brief description of the device

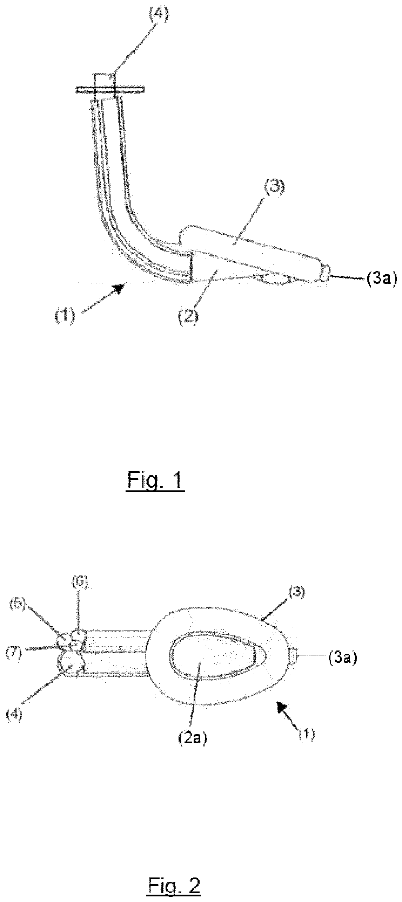

- Figure 1: shows a lateral view of the device (1), including the dome (2), the laryngeal ring (3) and the tubes (4) existing in this embodiment of the invention.
- Figure 2: shows an elevation view of the laryngeal ring (3) and the tubes existing in this embodiment of the invention, which in this case are all those mentioned above, i.e.: the oxygen tube (4) for allowing oxygen or gases to enter or leave the patient's internal airways; the laryngeal aspiration tube (5) for allowing air and/or secretions to leave the patient's internal airways; the gastric aspiration tube (6), and the pressure-measuring tube (7).

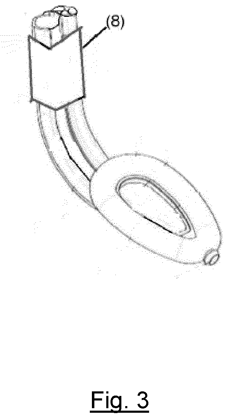

- Figure 3: shows an embodiment which comprises the additional piece (8) which surrounds the tubes and keeps them joined together, this piece additionally having the function of preventing the patient from biting the tubes and making them collapse.

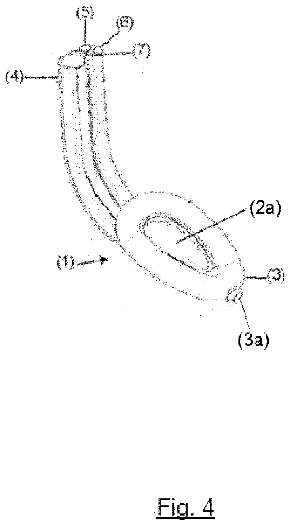

- Figure 4: shows a view in perspective of the laryngeal ring and the tubes mentioned above existing in this embodiment of the invention.

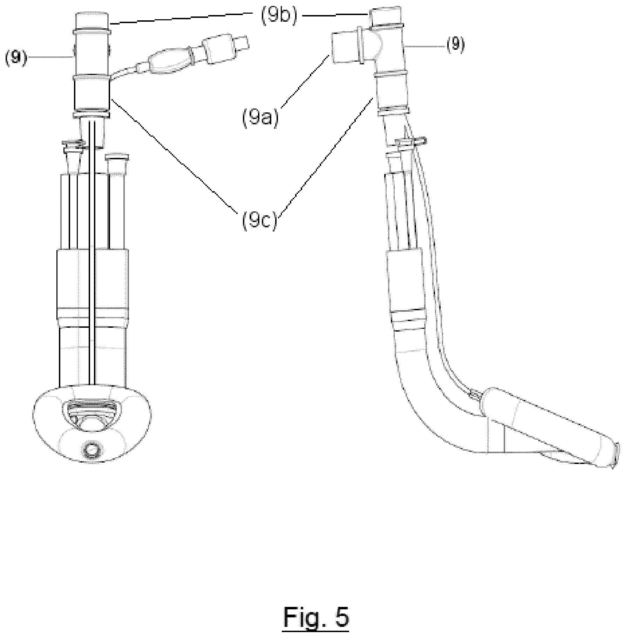

- Figure 5: shows an embodiment comprising the elbow piece (9) having three openings, one for the entrance of the continuous gas or oxygen flow supply (9a), other (9b) for the introduction of an extra accessory like a fiberscope, a bogie, a suction catheter or an echocardiograph, and another (9c) for the connection to the oxygen tube (4) of our laryngeal mask

Thus, the mask can be used in two ventilation modes. In either mode, the dome and the laryngeal ring are firstly introduced into the larynx of the patient. Once introduced, the laryngeal ring is inflated if required in order to seal the patient's larynx. Then, either of the following two operation modes are applied, depending on the ventilation mode chosen by the doctor or practitioner:
- In the standard or conventional ventilation mode, in which the device is used as a conventional laryngeal mask, the oxygen tube (4) will be open and connected to an external oxygen supply device such as an artificial respiratory machine or a ventilation bag. When in operation, through this tube the air or oxygen is alternatively forced into the internal airways of the patient, and also the breathed air is expelled out from the internal airways of the patient, thus making the function of a standard laryngeal mask working in standard or conventional ventilation mode. The laryngeal aspiration tube (5) will be plugged or closed in order to avoid interferences with the oxygen tube (4). The gastric aspiration tube (6) will be preferably open to facilitate aspiration of the gastric content and plugged to an aspiration device, and the pressure measuring tube (7) will be preferably plugged to a pressure measuring device.
- In the ventilation mode that supplies continuous oxygen positive pressure to the patient: in this ventilation mode the oxygen tube (4) will be open and connected to an external air and oxygen supply so that a supply of continuous or variable flow can be administered into the internal airways of the patient, and the laryngeal aspiration tube (5) will be open to a variable degree to allow the exhaled air leave outside the airways. The gastric aspiration tube (6) will be preferably open to allow the exit of the gas or gastric content in the patient's stomach and plugged to an aspiration device, and the pressure measuring tube (7) will be preferably connected to a pressure measuring gauge. In an example, such oxygen supply can be a continuous or variable flow of air or oxygen through a ventury device tap. In this way, a continuous positive pressure is kept residual in the internal airways of the patient. In this ventilation mode we can use an optional elbow piece (9) that has three openings, one for the entrance of the continuous gas or oxygen flow supply (9a), another (9b) for the introduction of an extra accessory like a fiberscope, a bogie, a suction catheter or an echocardiograph, and another opening (9c) for the connection to the oxygen tube (4) of our laryngeal mask. In this way, those extra accessories can be successfully operated according to their respective purpose while a supply of oxygen is continuously provided to the internal airways of the patient.

The mask of the invention can be used advantageously, for example:
- In patients with traumas, injuries, facial burns in which facial masks or the prongs used to administer continuous positive pressure placed on the patient's face is contraindicated;
- In patients with a beard or moustache, without teeth, macrologisa, an anatomical anomaly, goitre, and others, wherein facial masks or prongs used to administer continuous positive pressure placed on the patient's face are contraindicated because the mask does not seal properly and there are oxygen leaks on the face.
- In patients where it is dangerous that the continuous oxygen positive pressure supply administered with a mask face inflates the stomach of the patient, creating stomach dilatation, expansion, pain and risk of vomiting where we can develop a bronchoaspiration and a pneumonia with high mortality.

It is also useful when the positive pressure facial mask does not manage to recover the patient's normal level of oxygen saturation and there is a desire not to intubate, as well as when the patient is intolerant to the positive pressure facial mask and when it is not desirable to intubate the patient with sedation and relaxation drugs.

Also, the mask of the invention favours bronchodilation and reduces the resistance of the airway, re-expands atelectasis and allows the inspiration muscles to rest.

And it is useful when we want to keep a residual positive oxygen pressure on the dome of our laryngeal mask and on the patient airway when we want the patient to be asleep with spontaneous breathing and keep the airway reflexes.

## Claims

1. A laryngeal mask (1) for the ventilation of a human being or animal which comprises:
- a dome (2) having a cavity (2a), which dome (2) is attached to a laryngeal ring (3) for sealing the human being or animal's internal airways;
- at least one oxygen tube (4) that connects to the cavity (2a) of the dome (2), for allowing oxygen or gases to enter or leave the human being or animal's internal airways;
- at least one laryngeal aspiration tube (5), different from and situated side by side with the oxygen tube (4), that also connects to the cavity (2a) of the dome (2), for allowing the oxygen or gases entered into the human being or animal's internal airways through the oxygen tube (4) to leave the human being or animal's internal airways.
**characterised in that** the diameter of the laryngeal aspiration tube (5) is between 20 and 70% of the diameter of the oxygen tube (4).

2. A laryngeal mask according to claim 1, wherein the diameter of the laryngeal aspiration tube (5) is between 40 and 65% of the diameter of the oxygen tube (4).

3. A laryngeal mask according to claims 1 or 2, wherein the diameter of the laryngeal aspiration tube (5) is about 60% of the diameter of the oxygen tube (4).

4. A laryngeal mask according to claim 1, wherein the inner diameter of the laryngeal aspiration tube (5) is 5 mm and the inner diameter of the oxygen tube (4) is 8.5 mm.

5. A laryngeal mask according to any one of previous claims, which further comprises a gastric aspiration tube (6) that connects with the laryngeal tube orifice (3a) located at the distal portion of the laryngeal ring (3), for extracting excess air and/or secretions accumulated in the human being or animal's stomach.

6. A laryngeal mask according to any one of previous claims, which further comprises an additional tube (7) that connects to the cavity (2a) of the dome (2) and is connected on the opposite end to a pressure-measuring laryngeal mask, for measuring the pressure existing inside the dome.

7. A laryngeal mask according to any of the preceding claims which further comprises a protective piece (8) which surrounds the tube or tubes (4, 5, 6, 7) for protecting them from collapsing or biting and keeping them joined together.

8. A laryngeal mask according to any of the preceding claims, wherein the laryngeal ring is inflatable.

9. A laryngeal mask according to any of the preceding claims wherein the laryngeal ring is not inflatable.

10. A laryngeal mask according to any of the preceding claims which further comprises at least one cap or plug for the gradual or complete opening or complete closing of at least one of the tubes (4, 5, 6, 7) in any combination.

11. A laryngeal mask according to claim 10 wherein the cap or plug is a variable aperture plug and is adapted to close the laryngeal aspiration tube (5).

12. A laryngeal mask according to any previous claim further comprising an elbow piece (9) having three openings, wherein one of the openings (9a) is for the supply of oxygen, another opening (9b) is to allow the introduction of an extra accessory, and another opening (9c) to connect to the oxygen tube (4).

13. A laryngeal mask according to claim 12, wherein the extra accessory is selected from the group consisting of a fiberscope, a suction catheter, an echocardiograph and a bogie.

## Patentansprüche

1. Larynxmaske (1) zur Beatmung eines Menschen oder Tieres, umfassend:
- einen Gewölbekörper (2) mit einem Hohlraum (2a), wobei der Gewölbekörper (2) an einem Larynxring (3) zum Abdichten der inneren Atemwege des Menschen oder Tieres befestigt ist;
- mindestens einen Sauerstoffschlauch (4), der mit dem Hohlraum (2a) des Gewölbekörpers (2) verbunden ist, um zu ermöglichen, dass Sauerstoff oder Gase in die inneren Atemwege des Menschen oder Tieres eindringen oder aus ihnen austreten;
- mindestens einen Kehlkopf-Aspirationsschlauch (5), der sich von dem Sauerstoffschlauch (4) unterscheidet und neben diesem angeordnet ist, der auch mit dem Hohlraum (2a) des Gewölbekörpers (2) verbunden ist, um dem/den durch den Sauerstoffschlauch (4) in die inneren Atemwege des Menschen oder Tieres eingedrungenen Sauerstoff oder Gasen zu ermöglichen, die inneren Atemwege des Menschen oder Tieres zu verlassen.
**dadurch gekennzeichnet, dass** der Durchmesser des Kehlkopf-Aspirationsschlauches (5) zwischen 20 und 70 % des Durchmessers des Sauerstoffschlauches (4) beträgt.

2. Larynxmaske nach Anspruch 1, wobei der Durchmesser des Kehlkopf-Aspirationsschlauches (5) zwischen 40 und 65 % des Durchmessers des Sauerstoffschlauches (4) beträgt.

3. Larynxmaske nach einem der Ansprüche 1 oder 2, wobei der Durchmesser des Kehlkopf-Aspirationsschlauches (5) etwa 60 % des Durchmessers des Sauerstoffschlauches (4) beträgt.

4. Larynxmaske nach Anspruch 1, wobei der Innendurchmesser des Kehlkopf-Aspirationsschlauches (5) 5 mm und des Innendurchmessers des Sauerstoffschlauches (4) 8,5 mm beträgt.

5. Larynxmaske nach einem der vorhergehenden Ansprüche, die ferner einen Magen-Aspirationsschlauch (6) umfasst, der mit der Larynxschlauchöffnung (3a) verbunden ist, die sich am distalen Abschnitt des Larynxrings (3) befindet, um überschüssige Luft und/oder Sekrete, die sich im Magen des Menschen oder des Tieres angesammelt haben, abzusaugen.

6. Larynxmaske nach einem der vorhergehenden Ansprüche, die ferner einen zusätzlichen Schlauch (7) umfasst, der mit dem Hohlraum (2a) des Gewölbes (2) verbunden ist und am gegenüberliegenden Ende mit einer Druck messenden Larynxmaske verbunden ist, um den in dem Gewölbekörper vorhandenen Druck zu messen.

7. Larynxmaske nach einem der vorhergehenden Ansprüche, die ferner ein Schutzstück (8) umfasst, welches das Rohr oder die Rohre (4, 5, 6, 7) umgibt, um sie vor Kollabieren oder Beißen zu schützen und sie zusammenzuhalten.

8. Larynxmaske nach einem der vorhergehenden Ansprüche, wobei der Larynxring aufblasbar ist.

9. Larynxmaske nach einem der vorhergehenden Ansprüche, wobei der Larynxring nicht aufblasbar ist.

10. Larynxmaske nach einem der vorhergehenden Ansprüche, die ferner mindestens eine Kappe oder einen Stopfen zum allmählichen oder vollständigen Öffnen oder vollständigen Schließen mindestens eines der Rohre (4, 5, 6, 7) in beliebiger Kombination umfasst.

11. Larynxmaske nach Anspruch 10, wobei die Kappe oder der Stopfen ein Stopfen mit variabler Öffnung ist und zum Verschließen des Kehlkopf-Aspirationsschlauches (5) geeignet ist.

12. Larynxmaske nach einem der vorhergehenden Ansprüche, die ferner ein Winkelstück (9) mit drei Öffnungen umfasst, wobei eine der Öffnungen (9a) für die Zufuhr von Sauerstoff, eine weitere Öffnung (9b) für die Einführung eines zusätzlichen Zubehörs und eine weitere Öffnung (9c) für die Verbindung mit dem Sauerstoffschlauch (4) vorgesehen ist.

13. Larynxmaske nach Anspruch 12, wobei das zusätzliche Zubehör ausgewählt ist aus der Gruppe bestehend aus einem Fiberskop, einem Saugkatheter, einem Echokardiographen und einem Bougie.

## Revendications

1. Un masque laryngé (1) à des fins de ventilation d'un être humain ou d'un animal qui comprend :
- un dôme (2) ayant une cavité (2a), ledit dôme (2) étant fixé à un anneau laryngé (3) permettant d'étanchéifier les voies respiratoires internes de l'être humain ou de l'animal ;
- au moins un tube à oxygène (4) qui se connecte à la cavité (2a) du dôme (2), permettant à l'oxygène ou aux gaz de pénétrer ou de quitter les voies respiratoires internes de l'être humain ou de l'animal ;
- au moins un tube d'aspiration laryngé (5), différent du tube à oxygène (4) et situé aux côtés de ce dernier, qui se connecte également à la cavité (2a) du dôme (2), afin de permettre à l'oxygène ou aux gaz qui ont pénétré les voies respiratoires internes de l'être humain ou de l'animal via le tube à oxygène (4) de quitter les voies respiratoires internes de l'être humain ou de l'animal,
**caractérisé en ce que** le diamètre du tube d'aspiration laryngé (5) est compris entre 20 et 70 % du diamètre du tube à oxygène (4).

2. Un masque laryngé selon la revendication 1, dans lequel le diamètre du tube d'aspiration laryngé (5) est compris entre 40 et 65 % du diamètre du tube à oxygène (4).

3. Un masque laryngé selon la revendication 1 ou 2, dans lequel le diamètre du tube d'aspiration laryngé (5) s'élève à environ 60 % du diamètre du tube à oxygène (4).

4. Un masque laryngé selon la revendication 1, dans lequel le diamètre interne du tube d'aspiration laryngé (5) s'élève à 5 mm et le diamètre interne du tube à oxygène (4) s'élève à 8,5 mm.

5. Un masque laryngé selon l'une des revendications précédentes, qui comprend en outre un tube d'aspiration gastrique (6) qui se connecte à l'orifice du tube laryngé (3a) situé au niveau de la partie distale de l'anneau laryngé (3), afin d'extraire l'excédent d'air et/ou les sécrétions accumulées dans l'estomac de l'être humain ou de l'animal.

6. Un masque laryngé selon l'une des revendications précédentes, qui comprend en outre un tube supplémentaire (7) qui se connecte à la cavité (2a) du dôme (2) et est connecté au niveau de l'extrémité opposée à un masque laryngé mesurant la pression, afin de mesurer la pression à l'intérieur du dôme.

7. Un masque laryngé selon l'une des revendications précédentes, qui comprend en outre un élément protecteur (8) qui entoure le ou les tubes (4, 5, 6, 7) afin de les empêcher de tomber ou d'être mordus et afin qu'ils ne se désolidarisent pas.

8. Un masque laryngé selon l'une des revendications précédentes, dans lequel l'anneau laryngé est gonflable.

9. Un masque laryngé selon l'une des revendications précédentes, dans lequel l'anneau laryngé n'est pas gonflable.

10. Un masque laryngé selon l'une des revendications précédentes, qui comprend en outre au moins un couvercle ou un bouchon à des fins d'ouverture progressive ou complète ou de fermeture complète d'au moins l'un des tubes (4, 5, 6, 7) dans n'importe quelle combinaison.

11. Un masque laryngé selon la revendication 10, dans lequel le couvercle ou le bouchon est un bouchon à ouverture variable et est conçu de sorte à fermer le tube d'aspiration laryngé (5).

12. Un masque laryngé selon l'une des revendications précédentes, qui comprend en outre une pièce coudée (9) ayant trois ouvertures, dans lequel une des ouvertures (9a) assurant la fourniture d'oxygène, une autre ouverture (9b) permettant l'introduction d'un accessoire supplémentaire, et l'autre ouverture (9c) permettant de se connecter au tube d'oxygène (4).

13. Un masque laryngé selon la revendication 12, dans lequel l'accessoire supplémentaire est choisi dans le groupe constitué d'un fibroscope, d'un cathéter de succion, d'un échocardiographe et d'une sonde.
